# EUROPEAN PATENT APPLICATION

(11) **EP 2 857 520 A1**
(43) Date of publication of application: **08.04.2015**
(21) Application number: 13306378.4
(22) Date of filing: 03.10.2013
(51) Int. Cl.: C12Q 1/68

(54) **Method for in vitro investigating mitochondrial dna levels in a biological sample, kits and uses thereof**

(71) Applicant: INSTITUT PASTEUR, 75724 Paris Cedex 15 (FR); Instituto Mexicano Del Seguro Social, 06720 Mexico (MX); Centre National de la Recherche Scientifique, 75794 Paris Cedex 16 (FR)
(72) Inventor: Touati, Eliette, Camille, 92320 Chatillon (FR); Fernandes, Julien, 75019 Paris (FR); Michel, Valérie, 77330 Ozoir la Ferriere (FR); Torres Lopez, Javier, Mexico (MX)
(74) Representative: Desaix, Anne

(57) **Abstract**

The invention relates to an *in vitro* method for investigating the level of mtDNA in a biological sample removed from a patient susceptible of suffering from a gastric cancer condition or susceptible of suffering of condition(s) susceptible to evolve in a gastric cancer condition by pooling said biological sample into categories, upon determination of the level of mtDNA in said biological sample. The invention also relates to a method for *in vitro* monitoring or diagnosing the health status of a patient susceptible of suffering from a gastric cancer condition or suffering from a gastric cancer condition or susceptible of suffering of condition(s) susceptible to evolve in a gastric cancer condition, comprising a step of measuring, in parallel to the investigation of the level of mtDNA, the level of any one of the following parameters: cytokine IL-8, platelet count (PLT) and/or mean platelet volume (MPV) and/or percentage of large platelets (LPLT), inflammatory markers such as IL-6 and/or IL-23, or any combination of these parameters, and concluding about the health status of the tested patient. The invention also relates to kit(s) suitable for carrying out the method(s) of the invention and their use.

SEQUENCE LISTING

<110>  INSTITUT PASTEUR
       INSTITUTO MEXICANO DEL SEGURO SOCIAL
       CENTRE NATIONAL DE LA RECHERCHE SCIENTIFIQUE

<120>  METHOD FOR IN VITRO INVESTIGATING MITOCHONDRIAL DNA LEVELS IN A
       BIOLOGICAL SAMPLE, KITS AND USES THEREOF

<130>  B10233 - AD/AFL/KN

<140>  EP XXX XXX
<141>  2013-10-03

<160>  4

<170>  PatentIn version 3.5

<210>  1
<211>  20
<212>  DNA
<213>  Artificial Sequence

<220>
<223>  Forward primer hybridizing to the 12SRNA

<400>  1
gctcgccaga acactacgag                                                   20


<210>  2
<211>  21
<212>  DNA
<213>  Artificial Sequence

<220>
<223>  Reverse primer hybridizing to the 12SRNA

<400>  2
cagggtttgc tgaagatggc g                                                 21


<210>  3
<211>  20
<212>  DNA
<213>  Artificial Sequence

<220>
<223>  Forward primer hybridizing to the 18SRNA

<400>  3
gagaaacggc taccacatcc                                                   20


<210>  4
<211>  20
<212>  DNA
<213>  Artificial Sequence

<220>
<223>  Reverse primer hybridizing to the 18SRNA

<400>  4

gcctcgaaag agtcctgtat 20

## Description

The present invention relates to the field of *in vitro* testing methods based on the investigation of the level of mitochondrial DNA (mtDNA) in biological samples collected from individuals, animals or humans, and in particular relates to methods that can be applied to the monitoring and/or diagnosing of the health status of a patient susceptible of suffering from a gastric cancer or susceptible of suffering from condition(s) that may evolve in a gastric cancer, especially methods suitable for the monitoring or the detection of a risk of gastric carcinogenesis. The invention also relates to kits for performing the methods of the invention and their uses.

Gastric cancer (GC) is the second leading cause of cancer-related death in developing countries and the fourth in industrialized countries. It affects about one-million people per year.¹ Although the incidence of GC and mortality rates have gradually decreased in the last decades, it remains an important public health problem worldwide with approximately 800 000 deaths per year.

GC is often asymptomatic or causes only nonspecific symptoms in its early stages. By the time heavy symptoms occur, the cancer has often reached an advanced stage and may have also metastasized.

Actually and as a consequence, GC is often diagnosed at an advanced stage, e.g at stage IV for 79% of the tumors. These tumors generate early hematogenic metastasis to the liver, lung, brain and skeleton. Consequently, gastric cancer carries a poor prognosis with an overall 5-years survival rate around 15%.² However if it is diagnosed at an early stage, it can be a curable disease.³ These data highlight the importance and the need for characterization and validation of early GC biomarkers to reduce the morbidity and mortality associated to gastric adenocarcinoma.

GC arises from the complex interplay of various factors including environmental factors and host genetic factors.^{4, 5} However, the major risk factor occurring for 60 to 80% of gastric cancer cases is *Helicobacter pylori* infection. The prevalence of *H. pylori* infection is high, with 80 to 95% of the population infected in developing countries and up to 30% to 40% of adults in industrialized countries.⁶ The infection mostly persists life-long if not treated. All infected individuals develop a gastritis which can evolve to peptic ulcer diseases in 10% of the cases, while gastric adenocarcinoma and mucosa-associated lymphoid tissue (MALT) lymphoma will develop for 3% and 0.3% of infected subjects, respectively.^{6, 7}

Gastric adenocarcinoma is a malignant epithelial tumor, originating from glandular epithelium of the gastric mucosa. A major proportion of GC, i.e. more than 90%, are adenocarcinomas. Based on an histological distinction, there are two major types of gastric adenocarcinoma: intestinal type or diffuse type. Adenocarcinomas tend to aggressively invade the gastric wall, infiltrating the gastric mucosa, and present several degrees of differentiation, from well to poorly differentiated.

MALT lymphoma (or MALToma) is a form of lymphoma involving the mucosa-associated lymphoid tissue (MALT), frequently found in the stomach. This disease originates from B cells in the marginal zone of the MALT, and is also called extranodal marginal zone B cell lymphoma. Gastric MALT lymphoma is frequently associated (but not in all cases) with a chronic inflammation resulting from the presence of *H. pylori,* or linked with the presence of *H. pylori.*

In the context of the invention, GC encompasses gastric adenocarcinoma and MALT lymphoma.

GC of intestinal-type is mostly induced by *H. pylori* infection. It develops through a sequence of precursor lesions from atrophic gastritis to intestinal metaplasia (IM) then dysplasia and cancer.⁸ IM are recognized as pre-neoplastic lesions. It is important to notice that the eradication of the infection at an early stage (IM) can reverse and more importantly prevent the development of precancerous lesions.⁵ Accordingly, in humans several studies showed regression of precancerous lesions after *H. pylori* eradication,^{9 10} also confirmed in animal models. ^{11, 12}

In intestinal-type of GC, the sequential steps in the precancerous process can be considered as (in evolving order): gastritis (superficial or, at a later stage, chronic atrophic gastritis, for example), metaplasia (small intestinal or colonic metaplasia), and finally, dysplasia.

The cancerous (or neoplasic) stage may then be entered, depending on the evolution of the disease. The clinical stages of GC can be classified using known classifications systems, e.g. the TNM Classification of Malignant Tumours staging system that describes the extent of a patient's cancer. Using this type of classification, one can distinguish between Stages 0, I, II, III or IV. Other types of staging systems can be used.

In Stage 0, the gastric cancer is limited to the inner lining of the gastric mucosa and may be treatable by surgery when found very early, without need for chemotherapy or radiation treatments.

In Stages I and II, the disease has penetrated the deeper layers of the gastric mucosa, and may be treated by surgery, sometimes associated with chemotherapy and/or radiation treatments.

In Stage III, the disease may have penetrated other nearby tissues distant lymph nodes. Treated as for Stage II, a cure is still possible in some cases.

In Stage IV, the disease has spread to nearby tissues and more distant lymph nodes, or has metastasized to other organs. A cure is very rarely possible at this stage. Some techniques are used to prolong life or improve symptoms, including laser treatment, surgery, and/or stents to keep the digestive tract open, and chemotherapy.

Mitochondria are essential organelles of eukaryotic cells and possess their own genome. Defects in mitochondrial functions are associated with various human diseases including cancer.¹³ In humans, mitochondrial DNA (mtDNA) is a 16.6Kb circular DNA molecule present at hundreds to thousands copies per cell.¹⁴ Mutations in mtDNA have been reported in all cancer examined to date. As example in colorectal cancer, presence of mtDNA mutation is associated with a poor prognosis.¹⁵ The presence of mtDNA mutations has also been described at early stages of gastric carcinogenesis.^{16, 17} In *H. pylori* chronic gastritis patients, mtDNA mutations are significantly more frequent in gastric cancer patients than in cancer free patients.¹⁸ According to the inventors previous studies, mtDNA mutations are induced *in vitro* in *H. pylori* infected gastric epithelial cell¹⁹ and in the gastric mucosa of mice chronically infected.²⁰ Tumor specific changes in mtDNA copy number have also been reported in a broad range of primary human cancers.^{21, 22}. Xia et al, reported mtDNA depletion in the peripheral blood of patients strongly associated with stage I breast tumors.²⁸ A decrease in mtDNA content has been described in most tumor tissues of advanced GC, compared with nearby non-tumorous control tissue.²³ Variation of mtDNA level in cancer patients compared to healthy subjects has also been described for circulating cell-free mtDNA in the plasma and serum, leading to postulate that level of circulating mtDNA can constitute a potential preventive/diagnosis cancer marker detectable by a non-invasive method.¹⁹ Indeed, changes in mtDNA content in circulating blood samples have been described in patients with various types of tumors. As example, levels of circulating mtDNA were significantly higher in patients with urologic malignancies compared to healthy volunteers,²⁴ as also reported in several case-control studies in breast,²⁵ colorectal²⁶ and lung cancer.²⁷ These studies indicate that increased mtDNA content in peripheral blood should be associated with elevated cancer risk. In a recent case-control study conducted within a large prospective cohort of women residing in Shangai, no association was shown between leukocyte mtDNA copy number and presence of gastric tumor.²⁹ However a positive association between low mtDNA copy number in blood drawn within the two years prior to cancer diagnosis and risk of developing GC was observed, suggesting that circulating blood mtDNA levels could be an indicator of the presence of GC lesions at an early stage.

So far, variation of mtDNA levels in circulating blood can therefore be considered as a potential marker for the development of serological diagnosis test for a detection of the presence of gastric preneoplasia or cancer lesions.

However, the literature is not consistent when it comes to determining what variation(s) in mtDNA levels may be correlated with the presence of gastric preneoplasia or cancer lesions associated with a risk of gastric cancer, let alone what particular mtDNA levels may be associated with such a risk.

The present invention proposes a solution to this uncertainty, and further provides tools for investigating the level of mtDNA in a biological sample, in particular in a blood sample, said sample being obtained from a patient susceptible of suffering from a gastric cancer condition or condition(s) susceptible to evolve in a gastric cancer, in particular for monitoring or detecting a risk of an ongoing gastric carcinogenesis process.

As used herein, the term "*gastric carcinogenesis*" encompasses both the precancerous manifestations described above including gastritis, pre-neoplastic lesions such as intestinal metaplasia (small intestinal or colonic metaplasia), neoplastic lesions such as dysplasia, that may evolve in gastric cancer, and the different cancerous stages of gastric cancer, in particular, use of the expression "*gastric carcinogenesis*" covers the development of gastric cancer since detection of the presence of a gastritis or associated lesions in a patient.

The method of the invention can suitably be used for monitoring or diagnosing the health status of a such a patient.

The present invention relies on studies in which the peripheral leucocytes mtDNA levels have been measured in patients at various steps of the gastric carcinogenesis process. Relevant variations of mtDNA levels have been surprisingly found during the progression from gastric inflammatory lesions in gastritis patients until the development of malignant lesions, supporting the notion that circulating mtDNA level can be considered as a potential biomarker for detection of gastric carcinogenesis, in particular at early steps during gastric carcinogenesis.

Accordingly, the present invention relates to an *in vitro* method for investigating the level of mtDNA in a biological sample removed from a patient susceptible of suffering from a gastric cancer condition or susceptible of suffering of condition(s) susceptible to evolve in a gastric cancer condition, by pooling said biological sample removed from such a patient into categories, said method comprising the steps of:
a. Determining the level of mtDNA in said biological sample, and
b. Comparing the level of mtDNA determined in step a. with a normal threshold value determined for healthy subjects, and
c. From the comparison made in step b., assigning the tested biological sample to one of the following categories:
   - Group I: if the level of mtDNA determined in step a. is decreased with respect to the normal threshold value introduced in step b. by less than 2 folds, i.e., the ratio of the level of mtDNA determined in step a. over the normal threshold value determined for healthy subjects of step b. is inferior to 2,
   - Group II: if the level of mtDNA determined in step a. is from 2 and 20 folds with respect to the normal threshold value introduced in step b., i.e., the ratio of the level of mtDNA determined in step a. over the normal threshold value determined for healthy subjects of step b. is equal or superior to 2, but equal or inferior to 20,
   - Group III: if the level of mtDNA determined in step a. is increased with respect to the normal threshold value introduced in step b. by more than 20 folds, i.e., the ratio of the level of mtDNA determined in step a. over the normal threshold value determined for a pool of healthy subjects of step b. is superior to 20.

According to a particular embodiment, a level of mtDNA as determined in step a., which enables the assignment of the tested biological sample to Group I or Group III, is an indicator for the presence, or indicates the possibility of presence, of gastritis lesions (in Group I) or gastric neoplasia (in Group III), leading to further clinical investigation for the subject(s) assigned to these two groups, including endoscopy and anatomopathology analysis.

By "*patient susceptible of suffering from a gastric cancer condition or susceptible of suffering of condition(s) susceptible to evolve in a gastric cancer condition*", it is meant a patient presenting a suspicion for developing gastric cancer, for example because of physical clues, family history or complaint(s) indicating to the practitioner that an etiology of gastric cancer may be present or may become present. It also includes patients with gastroesophageal reflux, with chronic gastric pain as well as *H. pylori* seropositive subjects or *H. pylori* seronegative subjects, which have been beforehand eradicated for *H. pylori* infection. This definition also encompasses individuals having a declared gastric cancer condition or condition(s) susceptible to evolve in a gastric cancer condition, under treatment or not, which should be monitored. A particular group of patients eligible for the performance of the method of the invention is a group of patients with chronic inflammation associated with gastritis, or patient(s) with gastroesophageal reflux, with chronic gastric pain as well as *H. pylori* seropositive subjects. As mentioned above, a "*patient susceptible of suffering from a gastric cancer condition or susceptible of suffering of condition(s) susceptible to evolve in a gastric cancer condition*" may also be *H. pylori* negative at the time of the sampling and/or testing because of a successful eradication of the infection. The above definition also includes patients previously diagnosed for chronic atrophic gastritis or other gastric lesion that need a clinical follow-up.

In a particular embodiment, the method of the invention may be carried out on a sample isolated (collected, removed) from an individual who has been previously diagnosed for a gastric cancer or for lesions that may lead to a gastric cancer (pre-neoplasic condition) and who, optionally, may have been subjected to a treatment, such as surgery and/or chemotherapy and/or radiations treatment, in particular an individual pertaining to the above-mentioned particular group(s) of patient(s).

Under "*gastric cancer*" are encompassed: gastric adenocarcinoma, both diffuse or intestinal types, and MALT lymphoma.

By "*investigating the level of mtDNA*" it is meant detecting, in particular quantifying a *"level of mtDNA",* e.g. a quantity of mtDNA in an assayed biological sample collected from an individual, animal or human, with respect to a quantity considered as a standard value, said level reflecting the abundance of mtDNA copies in said sample.

By *"level of mtDNA",* it is meant a result qualifying the change in mtDNA with respect to a standard or normal level and in particular a value aimed at quantifying or representing the amount, in particular the number of copies of mtDNA, in particular the absolute number of copies of mtDNA, in the tested biological sample, or a relative amount of mtDNA in the tested biological sample, in particular when normalized with respect to a quantity of nuclear DNA (nDNA) or another suitable reference also present in said biological sample, or with respect to normalizing genes or DNA sequences pertaining to nDNA. In a particular embodiment, the investigation according to the invention may thus be achieved without determining the absolute quantity of mtDNA in the sample but by evaluating the variation (increase of the level or the decrease of the level) of said mtDNA and assigning said variation to a range as defined above.

To obtain a *"level of mtDNA"* according to the present invention, use is made of techniques enabling nucleic acids quantification from a biological sample. Such technique enables the determination of the average concentration (or amount) of nucleic acids, i.e., DNA or RNA, especially mtDNA within the context of the present invention, present in a sample. Several methods can be used to establish such concentrations (or amounts), including (1) spectrophotometric analysis of nucleic acids and their further quantification and (2) quantification using the measurement of the fluorescence intensity of dyes that bind to nucleic acids and selectively fluoresce when bound, as well as (3) quantification after specific nucleic acids amplification, such as in the real time PCR technique, which also relies on the detection of a fluorescent dye bound to said nucleic acids to be detected and quantified. All these techniques may be used with their corresponding appropriate containers for the nucleic acids to quantify, such as cuvettes or microplates or arrays, as well as combined in more integrated systems such as chips, lab-on-a-chip, microfluidic supports (including microfluidic cartridge or chip). Depending on the technique used, prior isolation of mtDNA to be quantified may be required, according to the common knowledge in the art of nucleic acids analysis.

By "*a normal threshold value determined for healthy subjects*", it is meant a value found by assaying one biological sample from an healthy subject or alternatively found by assaying several biological samples from several distinct healthy subjects, the resulting normal threshold value being then determined as the mathematical mean of the levels values of all the assayed healthy subjects biological samples, or alternatively found by assaying a pool of biological samples from several distinct healthy subjects.

According to a particular embodiment, such a normal threshold value is determined for a pool of healthy subjects.

By "*healthy subject(s)*" it is meant subjects that would have no symptoms of gastric disorders or patients referred to for gastroscopy with gastric biopsies corresponding to a normal phenotype, and in both cases not infected with *H. pylori* or that received an eradication therapy followed by a confirmed negative *H. pylori* serology. In a particular embodiment, the biological sample removed from such healthy subject(s) is a blood sample.

More particularly, by "*normal threshold value determined for healthy subjects or for a pool of healthy subjects*", it is meant a value, corresponding to a level of mtDNA, which may be predetermined according to the knowledge of the skilled in the art because it is known to correspond to a normal mtDNA value, or, according to a preferred embodiment, a value determined from assays made on subjects, in particular individuals, known to be healthy, i.e., not presenting a gastric cancer condition or suffering of condition(s) susceptible to evolve in a gastric cancer, or as explained above, said patients providing samples which either are individually tested and the results obtained from said samples treated to provide a mean value or a median value or said samples are tested as pooled samples to provide a so-called normal value. The thus obtained value provides a threshold that determines the reference value for normal samples.

By "*fold*", it is meant the expression of a change, in particular a number, describing how much a given quantity changes from a normal to a tested value, the normal value being in particular the "normal threshold" and the tested value being the mtDNA copy number or a representative value thereof, in the tested sample. For example, a normal value of 30 and a tested value of 60 correspond to a fold change of 2, or in common terms, a two-fold increase. To the contrary, a normal value of 60 and a tested value of 30 correspond to a fold change of 0.5, or in common terms, a 0.5 fold decrease, also referred to as a "minus" two-folds decrease (expressed in negative terms, with a "minus" sign before the number). Fold changes therefore correspond to a ratio of the tested value to the normal value. In other words, the fold change results from the determination of a ratio of the tested value against the normal value.

The invention accordingly also relates to a method of determining a "normal threshold" value for the mtDNA which value is associated with an absence of gastric cancer condition or an absence of a condition susceptible to evolve in a gastric cancer condition as disclosed herein.

The determination of "normal threshold" value may be regarded as involving a pooling method to the extent that it results either from pooling the results or from pooling the samples said samples being obtained from healthy subjects. Healthy subjects refer to a subject with no diagnosed chronic inflammation either gastric or other and with a negative *H. pylori* serology. It can also be a subject that is not treated by antibiotherapy or Non-Steroidal Anti-Inflammatory Drug(s) (NSAID), with a normal blood count and in the absence of any medical treatment at the time of the sampling and/or testing. According to another particular embodiment the normal values providing the threshold to determine the level of change in mtDNA of tested biological sample are the result of the above disclosed pooling method when performed on a pre-determined relevant population, as also detailed above.

The method of the invention can subsequently be used for monitoring or diagnosing the health status of a patient susceptible of suffering from a gastric cancer condition or susceptible of suffering of condition(s) susceptible to evolve in a gastric cancer condition, such a method comprising steps a., b. and c. as disclosed above and further comprising the following step:
d. from the assignment to a Group I, II or III made in step c. as described above, concluding about the health status of a patient from which the tested biological sample has been removed.

By "*concluding about the health status*" it is meant concluding that the individual, the biological sample of which is tested, may present a risk of suffering of a gastric cancer or from condition(s) that may evolve in a gastric cancer, i.e., condition(s) indicating a risk that a gastric carcinogenesis is present in the tested individual.

In particular and according to a finding related to the present invention, assignment of a mtDNA level value in Group I or III in step c. as described above, provides the information that the patient from which the tested biological sample has been removed is susceptible of suffering from gastric lesions, which also suggests performance of further clinical investigations. As non-limitative examples, other investigations methods aimed at confirming or excluding the presence of as gastric carcinogenesis process may be optical gastroscopic examination, computed tomography (or CT) scanning of the abdomen, biopsies for histological examination, various blood tests, e.g., Complete Blood Count (CBC) to check for anemia.

Accordingly, it is another object of the invention to provide a method for monitoring or detecting a risk of gastric carcinogenesis in a patient, wherein said method comprises identifying whether the level of mtDNA of the assayed sample has a value that pertains to Group I or Group III, a risk of gastric carcinogenesis being present if the biological sample removed from said patient has such a value.

By "*a risk of gastric carcinogenesis*" it is meant a risk of presenting a gastric cancer condition or a risk of presenting condition(s) susceptible to evolve in a gastric cancer condition, which encompass:
- precancerous manifestations of gastric cancer, that may further evolve in gastric cancer, such as: gastritis or gastritis lesions (superficial or, at a later stage, chronic atrophic gastritis, for example), metaplasia (small intestinal or colonic metaplasia), and finally, dysplasia, and
- the different cancerous (or neoplasic) stages of gastric cancer, as defined above.

According to a particular finding, the method of the invention enables concluding to the existence of a risk of gastric carcinogenesis, in particular at an early stage, when the tested biological sample pertains to Group I and the mtDNA level change measured with respect to normal value is less than 0.5 fold.

In a particular embodiment, by "*early stage of gastric carcinogenesis*" it is meant a pre-neoplasic stage, as defined above, for example a gastritis stage.

According to another particular finding, the method of the invention enables to conclude to the existence of a risk of a gastric cancer, when the tested biological sample pertains to Group III and the mtDNA level change measured with respect to normal value is more than 30 folds.

According to a particular embodiment, the gastric carcinogenesis referred to herein is associated with an *Helicobacter pylori* infection or is an *Helicobacter pylori-*induced gastric carcinogenesis.

Accordingly, the invention also comprises a method of investigating the level of mtDNA in a sample removed from a patient, where the patient has been previously or is simultaneously or in parallel tested for infection by *Helicobacter pylori.* The detection of *H. pylori* may be performed on a fraction of the sample removed from the patient, in particular on the serum fraction of a blood sample by carrying out a step of detection of antigens specific for *H.pylori* infection. People infected by *H. pylori* have specific IgA and IgG antibodies that can be easily detectable. In addition, the search for the presence of CagA antigens can also confirm the presence of *H. pylori.* Another method to detect *H. pylori* is the ¹³C urea breath test, a non-invasive test with high sensitivity widely used in human medicine (Graham et al, 1987, Lancet, 1 : 1174-1177). This respiratory test allows an indirect measure of the *H*. *pylori*-associated urease activity. Presence of *H. pylori* can also be detected in stools by immunoassay indicating the presence of *H. pylori* antigens or by amplification of *H. pylori* DNA in particular by polymerase chain reaction (PCR) using specific primers for *H. pylori* genes sequences, which are available in the literature to one skilled in the art, and detection of the amplified DNA.

For detection of *H. pylori,* the blood sample may be prepared on the one hand to purify the cellular fraction of the blood sample, in particular the mononuclear cells or leukocytes containing the mtDNA to be assayed and on the other hand to collect the serum enabling the detection of *H.pylori* infection.

In a particular embodiment, the tested biological sample is obtained from a patient diagnosed with gastric carcinogenesis and under treatment for this condition or not, and/or a patient having an ongoing, treated or not, *Helicobacter pylori* infection, and/or a patient having antecedents of *Helicobacter pylori* infection(s), eradicated by prior or ongoing treatment or not, and/or an individual having gastric pain and/or a family history of gastric cancer.

The method for investigating the level of mtDNA in a biological sample according to the invention is performed using any relevant process for the quantification of nucleic acids, as in particular introduced above. It especially involves treating the sample to gain access to mononuclear cells, in particular leukocytes, and furthermore to provide access to nucleic acid, especially by extracting the nucleic acid, and optionally by separating the mtDNA from nuclear DNA. The method also involves using polynucleotides, in particular probes and/or oligonucleotides when they are complementary to a region of interest in the mtDNA, said polynucleotides optionally including a detectable label or marker (such as a fluorochrome or a radioelement) in order to allow quantitative detection of the mtDNA.

According to an embodiment of the invention, the method for investigating the level of mtDNA in a biological sample and optionally the method of determining a "normal threshold" value involve(s) a step of amplification of the mtDNA, possibly a step of differential amplification of the mtDNA with respect to the nuclear DNA.

According to a specific embodiment, the *"level of mtDNA"* is determined by quantitative polymerase chain reaction (q-PCR). Primers specific for the *12sRNA* mitochondrial gene may be used, although one skilled in the art can suitably choose other genes or sequences of the mitochondrial genome for implementing such a technique, following guidance available in the literature of this field with respect to this technique.

PCR (polymerase chain reaction) is a common method for amplifying DNA. In order to amplify small amounts of DNA, a DNA template, at least one pair of specific oligonucleotide primers, nucleotides (dATP, dCTP, dGTP, dUTP), a suitable buffer solution and a thermo stable DNA polymerase are required. A substance marked with a fluorophore is generally added to one reagent of this mixture in a thermal cycler that contains sensors for measuring the fluorescence of the fluorophore after it has been excited at the required wavelength allowing the generation rate to be measured for one or more specific products. Real-time PCR (q-PCR) is generally applied to the detection and quantification of DNA in samples to determine the presence and/or abundance of a particular DNA sequence in these samples. A measurement is made after each amplification cycle, which enables the quantification of the amplified product in real time.

Real-time PCR is performed by using a real-time PCR apparatus, and after each cycle, the levels of fluorescence are measured with a detector. Used dyed generally only fluoresce when bound to the DNA amplified through PCR, and the increase of fluorescence is detected, corresponding to increasing presence of the amplified products, at each amplification cycle.

Real-time PCR can be used to quantify nucleic acids by either relative quantification or absolute quantification. Absolute quantification gives the exact number of target DNA molecules by comparison with DNA standards using a calibration curve. By this method, it is possible to determine the number of mtDNA copies in patients suspected for the presence of gastric pre-neoplasia or neoplasia and compare this number to the number of mtDNA copies defined in healthy subjects. (Ref: von Wurmb-Schwark et al, 2002, Forensic Science International, 126: 34-39). Relative quantification enables determining fold-differences between a target sequence, the quantity of which is to be determined, and a "housekeeping sequence".

In order to quantify the presence of a specific DNA target sequence, representative of the copy number of the mtDNA, it is indeed convenient to express its relative level in relation to another DNA sequence called a "normalizing sequence" or "housekeeping sequence", which is selected for its almost constant rate of expression. Housekeeping sequences are usually found in genes involved in the functions related to basic cellular survival, which normally implies constitutive gene expression. This enables the provision of a ratio expressing the presence of the amplified sequence of interest over the presence of the amplified selected normalizer. This method allows obtaining a value evaluating the relative presence of the amplified sequence of interest actually knowing its absolute quantity within the tested sample.

Commonly used normalizing sequences are those found in genes coding for the following proteins. As a non-limitative list, tubulin, glyceraldehyde-3-phosphate dehydrogenase, albumin, cyclophilin, ribosomal RNAs sequences can be used.

Real time PCR allows quantification of the desired product at any point in the amplification process by measuring fluorescence. Measurement is expressed using a Cycle Threshold (C_{T}) value (C_{T}; PCR cycle at which the fluorescence of the sequence of interest is detected; the lowest is the C_{T} value, the more abundant is the target sequence). To quantify the presence of the target sequence when a normalization sequence is used, a normalization procedure such as the ΔΔC_{T}-method can be used, said ΔΔC_{T}-method being used for analyzing a relative gene expression.

According to a more specific embodiment of the invention, the "*level of mtDNA*"i is determined by quantitative polymerase chain reaction (q-PCR) through reference to a selected normalizer gene or nDNA sequence, the level of mtDNA being calculated according to the formula 2^{ΔCt}, wherein ΔCt = Ct_{nDNA} - Ct_{mtDNA}, as described in reference publications Fan et al, 2009 , J Cancer Res Clin Oncol, 135 ; 983-989 and/or Chatre and Richetti, 2013, J of Cell Sciences, 126 : 914-926. With this calculation method, the level of mtDNA can be calculated using the ΔC_{T} of average C_{T} of mtDNA and nDNA (ΔC_{T} = Ct_{nDNA} - Ct_{mtDNA},) as 2^{ΔCt}. The primers used for amplification can be chosen by one skilled in the art according to the common knowledge in the field of this technique, as indicated in particular in the above-mentioned reference publications.

The invention thus concerns a method wherein the level of mtDNA is determined by quantitative polymerase chain reaction (q-PCR) following the steps of:
- preparing the biological sample to provide access to the nucleic acid, especially mitochondrial nucleic acid of cells;
- contacting the prepared sample with oligonucleotide primers targeting the mtDNA;
- performing amplification cycles,
- simultaneously running amplification of a normalizer nDNA,
- quantitatively detecting the mtDNA and the normalizer nDNA.
- determining the level of mtDNA through reference to a selected normalizer nDNA sequence, the level of mtDNA being calculated according to the formula 2^{ΔCt}, wherein ΔCt = Ct_{nDNA} - Ct_{mtDNA}.

The invention thus concerns a method for investigating the level of mtDNA in a biological sample wherein the biological sample is from a patient diagnosed with gastric carcinogenesis and under treatment for this condition or not, and/or a patient having an ongoing, treated or not, *Helicobacter pylori* infection, and/or a patient having antecedents of *Helicobacter pylori* infection(s), eradicated or not, and/or an individual having gastric pain and/or a family history of gastric cancer.

According to a particular embodiment, the mtDNA of the tested sample is detected or quantified by using a region in the 12sRNA gene of the mitochondrial genome, and the nDNA of the tested sample is detected or quantified by using a region in the 18sRNA gene of the nuclear genome. Other couples of mtDNA/nDNA primers (probes) can be chosen and used by the person skilled in the art, as reminded above, for example mtDNA encoded ATPase 8 gene and nDNA glyceraldehyde-3-phosphodehydrogenase (GAPDH) or Beta 2 microglobuline (β2M).

Accordingly, in a particular embodiment, oligonucleotide primers and/or DNA probes suitable for carrying out the invention may advantageously be selected for their capacity to hybridize to a strand of the 12sRNA gene or to a strand of the 18sRNA strand. The oligonucleotide (forward and reverse) primers have a length adapted to specifically priming the targeted mtDNA or nDNA and in particular have a length within a range of 10 to 30 nucleotides. Examples of oligonucleotide primers are polynucleotides comprising or consisting of the sequences disclosed in the experiments provided herein. The probes may have a length of 50 to several thousand of nucleotides, in particular may have a length of 50 to 500 nucleotides.

Oligonucleotides and probes to carry out the detection of mtDNA are selected for their ability to hybridize specifically with the targeted mtDNA and accordingly would not hybridize significantly in PCR operating conditions with nuclear DNA. Preferably, the chosen mtDNA sequences are highly specific to mitochondrial genome and nonexistent in nuclear genome.

The invention thus also relates to the use of the pairs of oligonucleotides either as unique pair or combined primer pairs having the following sequences hybridizing to the 12SRNA: 12S (forward): 5'- GCT CGC CAG AAC ACT ACG AG; (reverse): 5'- CAG GGT TTG CTG AAG ATG GCG for the detection of mtDNA. It may further involve primers as the following hybridizing with a nuclear encoded 18SRNA: 18S (forward): 5'- GAG AAA CGG CTA CCA CAT CC; (reverse): 5'- GCC TCG AAA GAG TCC TGT AT.

In a particular embodiment, the method of the invention is performed using a biological sample removed from a patient, that is a blood sample or, in particular when the patient already present gastric mucosa lesions, a biopsy sample, in particular a biopsy sample of gastric mucosa. All layers of the gastric mucosa may be used for sampling. mtDNA and/or DNA extraction and isolation can be performed according to commonly known methods.

According to another particular embodiment, the level of mtDNA is determined by testing circulating blood mtDNA, in particular is determined by testing the mtDNA of leukocyte(s), in particular leukocyte(s) isolated from a blood sample, especially when circulating blood samples are used for performing the method of the invention.

In a particular embodiment of the invention, the investigation of the level of the mtDNA in a patient is associated with the determination of a different biological parameter measured in order to improve the significance or the interpretation of the results obtained in relation with the determination of mtDNA level with a view to ascertain the patient's condition. Other biological parameters in this regard include the parameters which are known to provide indication as to a condition susceptible to evolve toward gastric carcinogenesis, such as measurement of cytokine, especially interleukin expression in particular pro-inflammatory interleukin, including IL-8 expression.

Another object of the invention is accordingly a method for monitoring or diagnosing the health status of a patient susceptible of suffering from a gastric cancer condition or susceptible of suffering of condition(s) susceptible to evolve in a gastric cancer condition, said method comprising the following steps:
a. Performing a method of the invention for investigating the level of mtDNA in the tested patient as described above, and
b. Measuring, in parallel to the investigation of the level of mtDNA of step a., the level of cytokine IL-8 in a biological sample removed from the patient tested in step a., and
c. Concluding about the health status of a patient on the basis of the results obtained from step a. and b.

By "*measuring, in parallel to the investigation of the level of mtDNA*", it is meant that the measurement of the level of cytokine IL-8 is made by using samplings of biological material(s) in a same individual, collected within a close interval of time, i.e. avoiding an interval of time that may render both parameters not representative of the physiological status of the individual at a given time, in particular for evaluating both parameters at a same given time t.

The inventors have indeed assessed the pertinence of measuring the level of cytokine IL-8 for a same tested individual, in particular for providing a better evaluation of the health status of said tested individual. A strong association between cancer and inflammation has long been observed. Association between circulating cytokine level(s) and gastric cancer risk has also been reported, highlighting in particular that an high *H. pylori* prevalence and increased circulating IL-8 level may be associated with increased risk of gastric cancer (Epplein et al, 2013, Cancer Causes Control, DOI: 10.107/s10552-013-0284-z).

Levels of cytokine IL-8 can be measured using commonly known methods in the art such as, as a non limitative example, through enzyme-linked immunosorbent assay(s) (ELISA) (Engvall E and Perlman P, 1971, lmmunochemistry, 8: 871-874). For example, levels of cytokine IL-8 can be measured using the ELISA kit commercialized by BD Biosciences: BDOptEIA Set Human IL-8. Cat. No. 555244.

According to a particular embodiment, if one determines that the level of mtDNA pertains to Group I or Group II and the level of cytokine IL-8 is above 100 pg/mL, conclusion can be made of a risk of presence of a gastric cancer.

In another aspect, the invention also relates to a method for monitoring or diagnosing the health status of a patient susceptible of suffering from a gastric cancer condition or suffering from a gastric cancer condition or susceptible of suffering of condition(s) susceptible to evolve in a gastric cancer condition, said method comprising the following steps:
a. Performing a method of the invention for investigating the level of mtDNA in the tested patient as described above, and
b. Measuring, in parallel to the investigation of the level of mtDNA of step a., the level of any one of the following parameters: cytokine IL-8, platelet count (PLT) and/or mean platelet volume (MPV) and/or percentage of large platelets (LPLT), inflammatory markers such as IL-6 and/or IL-23, or any combination of these parameters in a biological sample removed from the patient tested in step a., and
c. Concluding about the health status of a patient on the basis of the results obtained from step a. and b.

Matowicka-Karna J et al (Volume 2013, article ID 401623, Clinical and Developmental Immunology) have evidenced the contribution of platelets and inflammatory markers in gastric cancer. Generally cancer cells are a source of inflammatory cytokines and growth factors such as IL-6 or IL-23. IL-6 and IL-23 are actively involved in the pathogenesis and development of tumors. They facilitate tumor growth by inhibiting apoptosis of tumor cells and by angiogenesis induction within the tumor. IL-6 is a pleiotropic cytokine involved in immune responses, inflammatory reactions, and haematopoiesis. A direct association between increasing plasma levels of IL-6 and gastric cancer risk has been reported in Wong et al, 2011, Cancer Sciences, 102: 1911-1915. IL-23 strongly induces the secretion of IFN-γ, affects the haematopoiesis by stimulating thrombocytopoiesis, and the production of neutrophils, and stimulates the production of acute phase proteins. Platelets take part in inflammation and in cancerous diseases.They play an active role in the inflammatory process due to secreted proinflammatory factors, chemokines, and growth factors. An excessive number of platelets is the cause of a significant increase in the risk of metastases in each stage of cancer and an indicator of poor prognosis, for example, in gastric, lung, and kidney cancer. In cancer disease, an increase in the percentage of large platelets (LPLT) is observed, and because young, metabolically active platelets appear in the circulation, this may lead to an increase in MPV (mean platelet volume).

"Platelet count" (PLT) can be expressed as platelet count per unit volume of blood, generally ranging between 150.10⁹ and 350.10⁹ per liter of blood.

"Mean platelet volume" (MPV) can be calculated on the basis of distribution volume, generally ranging from 7,8 to 11,5 femtoliter (fl).

"Large platelets" are defined as platelets having a volume above 20 femtoliter (fl).

PLT, MPV and LPLT parameters can be collected through an hematology analyzer instrument, available in the art.

Levels (or concentrations) of IL-6 and/or IL-23 parameters can be measured using commonly known methods in the art, in particular through ELISA testing.

The above-mentioned parameters may also enable distinguishing between different gastric cancer stages. Reference is made to Matowicka-Karna J et al (Volume 2013, article ID 401623, Clinical and Developmental Immunology). In particular, high levels of IL-23 and IL-6 have been observed in patients with early gastric cancer. In the experiments described by Matowicka-Karna et al, a control level of IL-23 in a group of normal subjects was found to be 5.21±3.65 pg/ml compared to 20.42±1.44 pg/ml in a group of early gastric cancer patients. Control level for IL-6 was found to be 2.45±1.44 pg/ml in a group of normal subjects compared to 10.80±8.24 pg/ml in a group of early gastric cancer patients.

According to a particular embodiment, the method for monitoring or diagnosing the health status of a patient susceptible of suffering from a gastric cancer condition or suffering from a gastric cancer condition or susceptible of suffering of condition(s) susceptible to evolve in a gastric cancer condition of the invention encompasses a determination of any combination of the above-mentioned parameters, in particular a combination of determination of the level of mtDNA and the level of at least one of the parameters selected amongst: IL-8, PLT, MVP, LPLT, IL-6, IL-23, or a combination of determination of the level of mtDNA and the level of IL-8 and the level of at least one of the parameters selected amongst: PLT, MVP, LPLT, IL-6, IL-23, in particular a combination of determination of the level of mtDNA and the level of IL-8 and the level of any one of either IL-6 or IL-23.

The invention therefore also relates to a method for *in vitro* monitoring or diagnosing the health status of a patient susceptible of suffering from a gastric cancer condition or suffering from a gastric cancer condition or susceptible of suffering of condition(s) susceptible to evolve in a gastric cancer condition, said method comprising the following steps:
a. Performing a method of the invention as described herein for investigating the level of mtDNA in the tested patient, and
b. Measuring, in parallel to the investigation of the level of mtDNA of step a., the level of any one of the following parameters: cytokine IL-8, platelet count (PLT) and/or mean platelet volume (MPV) and/or percentage of large platelets (LPLT), inflammatory markers such as IL-6 and/or IL-23, or any combination of these parameters in a biological sample removed from the patient tested in step a., and
c. Concluding about the health status of a patient on the basis of the results obtained from step a. and b.

According to a particular embodiment, such a step c. of conclusion about the health status of a patient takes into account the value(s) of a level of IL-23 and/or of a level of IL-6 for detecting a risk of presence of a gastric cancer condition or the presence of a gastric cancer condition, in particular a gastric cancer condition at an early stage, in a tested patient, or takes into account a relevant increase of any one or all of such value(s) with respect to a normal threshold value determined for healthy subjects, as defined herein and according to the data provided herein and/or by Matowicka-Karna et al. An increase may be calculated in fold(s) for quantifying said increase.

According to a specific embodiment, conclusion is made of a risk of presence of a gastric cancer condition or conclusion is made of the presence of a gastric cancer condition, in particular a gastric cancer condition at an early stage, when the IL-23 level found in the tested sample(s) is superior to about 18 or about 19 or about 20 pg/ml, in particular superior to 20,5 pg/ml, and/or when the IL-6 level is superior to about 8 or about 9 or about 10 pg/ml, in particular superior to 10,8 pg/ml.

According to another specific embodiment, such a conclusion is made in combination with deduction(s) made with respect to another parameter, as described herein.

Another object of the invention is to provide a kit suitable for carrying out a method of the invention as defined herein, comprising:
- At least one pair of specific oligonucleotide primers specific for hybridization with mtDNA and, optionally, at least one pair of specific oligonucleotide primers specific for hybridization with H. pylori nucleic acid(s) sequence(s), and, optionally, one or several of the following reagents,
- nucleotides (e.g. dATP, dCTP, dGTP, dUTP),
- a DNA polymerase, in particular a thermostable DNA polymerase, such as a Taq DNA Polymerase,
- at least one dye for staining nucleic acids, in particular a dye detectable in a real-time PCT equipment,
- optionally, a buffer solution,
- optionally, reagents necessary for the hybridation of the primers to their targets,
- optionally, a reference dye and,
- optionally a notice providing instructions for use and expected values for interpretation of results.

According to a particular embodiment, a kit of the invention comprises primers suitable for amplifying 12S and optionally 18S gene sequences or fragments thereof, such as 5'- GCT CGC CAG AAC ACT ACG AG and 5'-CAG GGT TTG CTG AAG ATG GCG nucleotide sequences (12S), and/or 5'- GAG AAA CGG CTA CCA CAT CC and 5'- GCC TCG AAA GAG TCC TGT AT sequences (18S).

Other primers may be used within such a kit, as detailed hereabove.

Another object of the invention is to provide a kit suitable for carrying out a method of the invention as defined herein, comprising:
- at least one antibody specific for a protein selected amongst: IL-8, IL-6, IL-23 or a combination of several antibodies specific for IL-8, IL-6, IL-23, and, optionally, at least one antibody specific for *H. pylori* antigen(s), such as CagA antigens, and, optionally, one or several of the following reagents,
- a secondary antibody or reagent to reveal a complex between specific antibody(ies) recited above and its(their) target,
- optionally, a buffer solution,
- optionally, an assay plate, and
- optionally a notice providing instructions for use and expected values for interpretation of results.

The invention also relates to the use of kit(s) according to the invention for investigating the level of mtDNA or of the other parameter(s) described herein in a biological sample, and/or monitoring or diagnosing the health status of a patient susceptible of suffering from a gastric cancer condition or susceptible of suffering of condition(s) susceptible to evolve in a gastric cancer condition and/or monitoring or detecting a risk of gastric carcinogenesis, or suffering from a gastric cancer condition, including the health status of said patient with respect to an *H. pylori* infection, according to the description provided herein, in all its detailed or encompassed embodiments.

The present invention is a basis for a non-invasive test for measuring mtDNA levels, in particular in circulating blood. According to the invention mtDNA levels may be used as an efficient biomarker for an early detection of gastric carcinogenesis, in particular an early detection of the presence of gastric lesions involved or at the basis of a gastric carcinogenesis process. The present invention may be especially suitable for prevention purposes, but also for monitoring the progression of a gastric carcinogenesis process, subject to an on-going treatment or not, monitoring a shift from a pre-neoplasic condition to a neoplasic condition, or as a follow-up after a cure to screen for a recurrence of the disease.

The method of the invention may be used on patients under treatment, such as chemotherapy treatment and/or radiations treatment, as an indicator of treatment efficiency, disease stage, and disease development.

The method of the invention may be used on patients presenting a HER2 overexpressing gastric adenocarcinoma, treated or not with trastuzumab (Herceptin®, Genentech). Other known drugs used in gastric cancer chemotherapy are (non-limitative examples): 5-fluorouracil, cisplatin, epirubicin, etoposide, docetaxel, oxaliplatin, capecitabine, or irinotecan.

As a result of performing the methods of the invention on patients, other investigations methods aimed at confirming or excluding the presence of as gastric carcinogenesis process may be indicated, such as an optical gastroscopic examination, computed tomography or CT scanning of the abdomen, biopsies for histological examination, various blood tests may also be done, such as Complete Blood Count (CBC) to check for anemia.

The invention will be further described herein, referring to the following figures and experimental section.

### Legends of figures

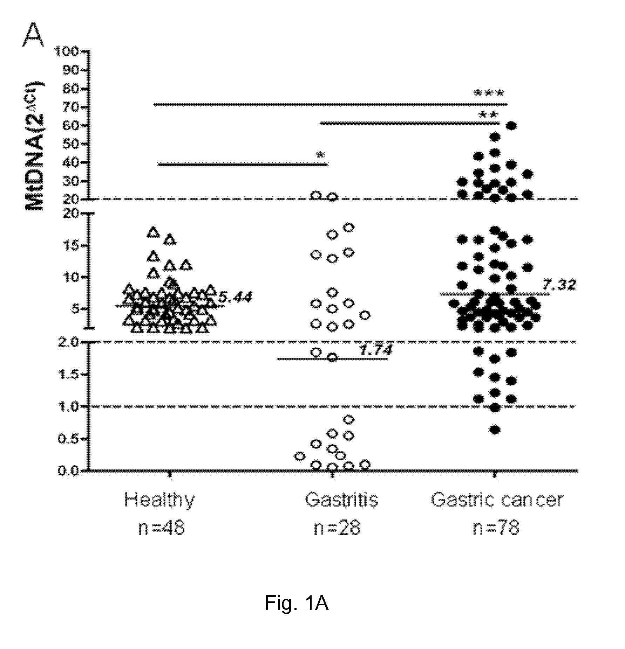

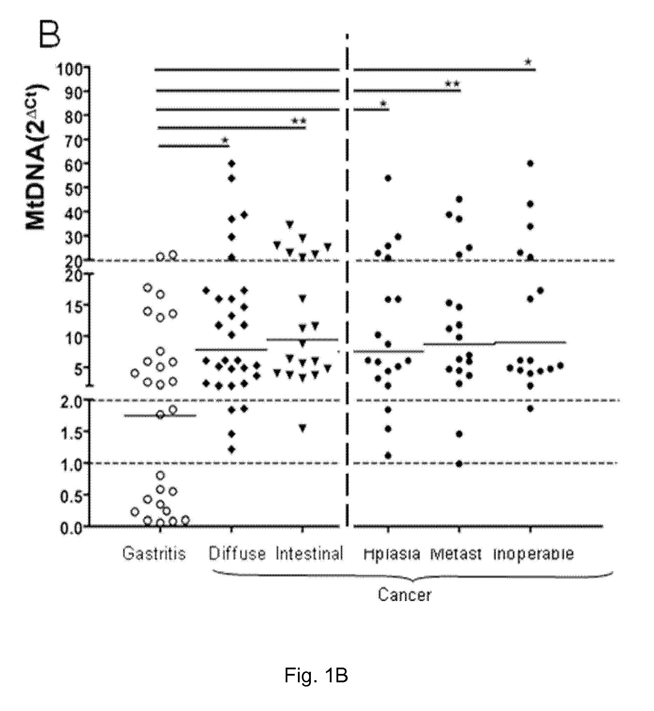

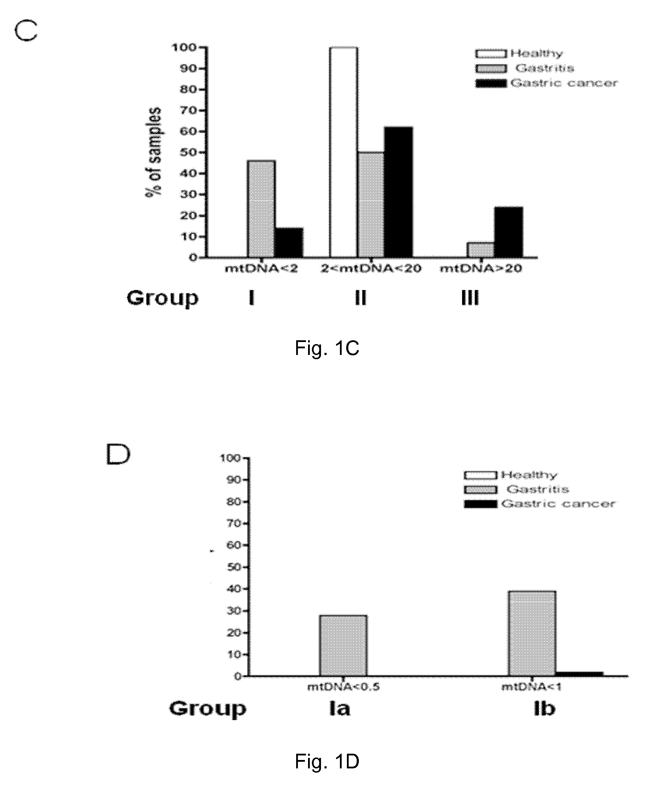

**Figure 1****. Circulating mtDNA content in white blood cells of NAG and GC patients compared to healthy blood donors. A)** MtDNA was quantified by quantitative real-time PCR from DNA isolated from peripheral leukocytes isolated from healthy blood donors *H. pylori*-negative (open triangles) and from non atrophic gastritis (NAG) (open circles) and GC patients (closed triangle) from cohort 1. MtDNA relative values was measured as previously described according to nuclear DNA (nDNA) (see Material and methods).³⁵ Each symbol corresponds to a single patient. Bars and numbers indicate geometric means. Data are obtained from measures done in triplicate in three independent experiments. **P*<0.05 ; ***P*<0.001. **B)** Peripheral leukocytes mtDNA level in NAG and GC patients according to the type of cancer either diffuse (n=31) or intestinal (n=20) or the presence of hyperplasia (n=19), metastasis (n=19) or late-stage cancer judged inoperable by surgeons (n=17) **C)** Distribution of the samples according to their mtDNA peripheral blood content in three different groups, Group I : mtDNA<2, Group II : 2<mtDNA<20 and Group III : mtDNA>20. No healthy samples are found in Group I. Samples with mtDNA level > 30 include only *H. pylori* infected gastric cancer patients. **D)** Samples with mtDNA<1 can subdivided in 2 sub-groups : Group la : mtDNA<0.5 corresponding exclusively to NAG samples and Group Ib : mtDNA<1 including 94% of NAG samples (16/17) and 6% of GC samples (1/17).

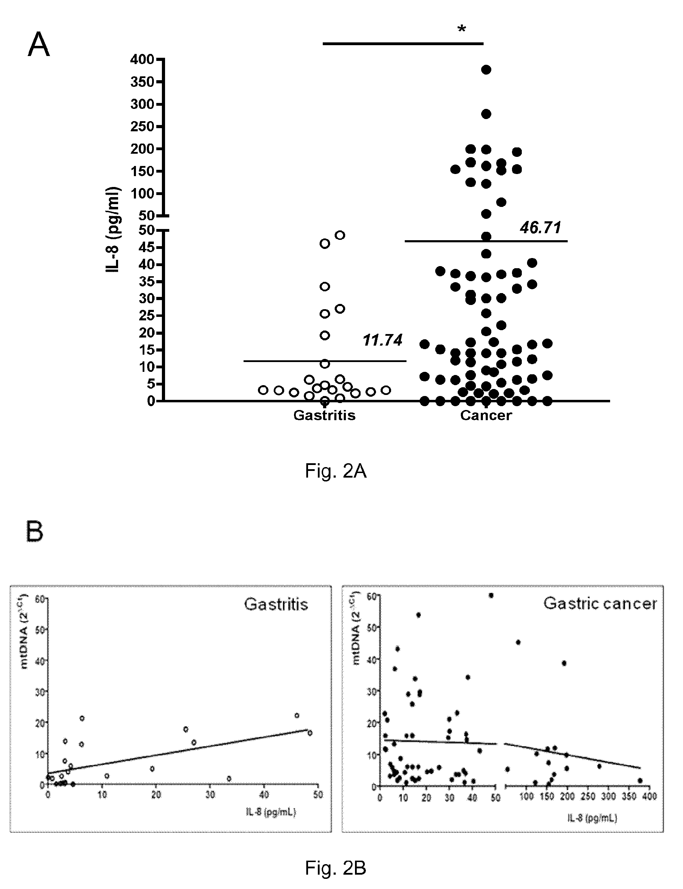

**Figure 2****. Comparison of IL-8 levels in sera of NAG and GC patients. A)** IL-8 was quantified by using the ELISA kit BDOptEIA Set Human IL-8. Cat. No. 555244 commercialized by BD Biosciences according to the recommendations of the fabricant, in the sera from NAG and GC patients from cohort 1. The mean IL-8 level is higher in GC (median= 3.95) than in NAG (median= 16.65) patients. **B)** Analysis of a correlation between IL-8 serological levels and peripheral leukocytes mtDNA amount in NAG and GC patients from cohort 1. A positive significant correlation between IL-8 and mtDNA was detected for NAG samples (r=0.586 ; p=0.0041) but not in the case of GC patients.

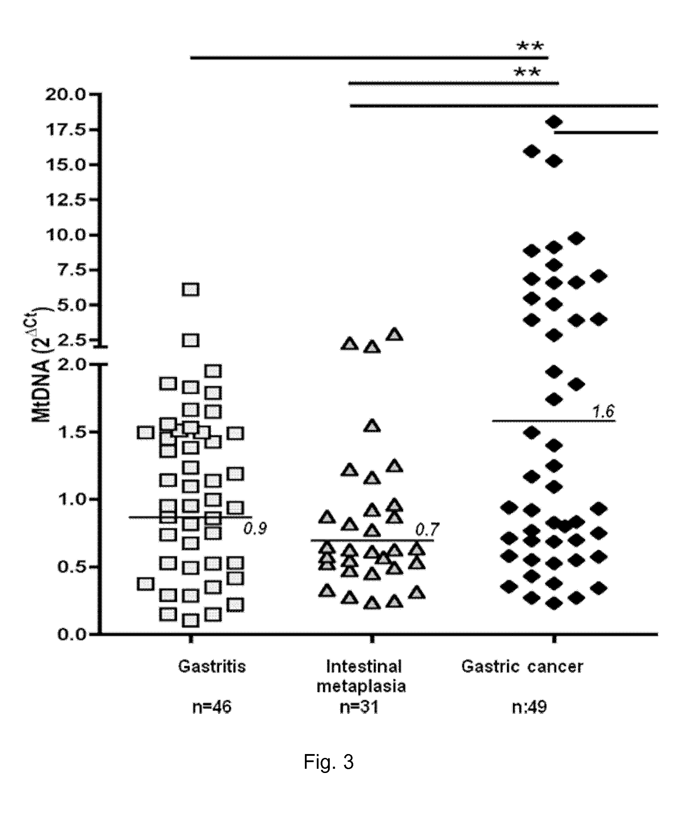

**Figure 3****. Comparison of mtDNA level at various steps of the gastric carcinogenesis process.** Quantification of peripheral leukocytes mtDNA level in NAG, IM and GC patients from cohort 2. MtDNA was measured as previously reported³⁵ and described in material and methods.

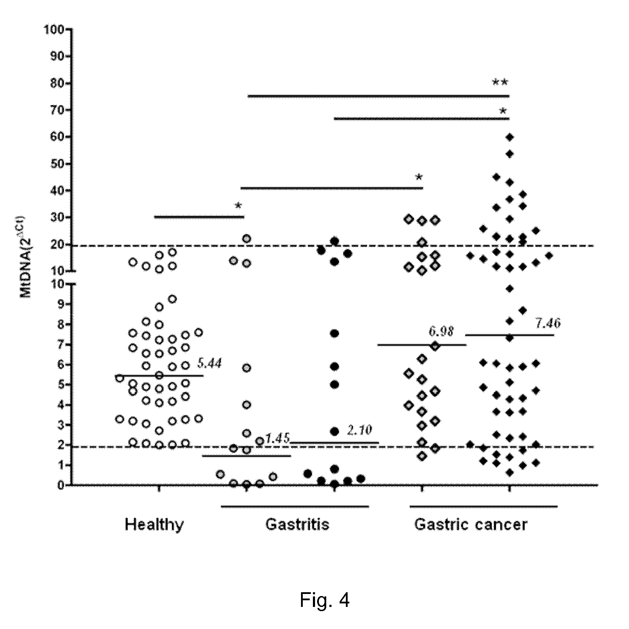

**Figure 4** **: Circulating mtDNA content in white blood cells of NAG and GC patients compared to healthy blood donors according to their *H*. *pylori* serology.** MtDNA was quantified by quantitative real-time PCR from DNA isolated from peripheral leukocytes isolated from healthy blood donors *H. pylori*-negative (open circles) and from non atrophic gastritis (NAG) *H. pylori* negative (grey circles) and *H. pylori* positive (closed circles) and GC patients *H. pylori* negative (grey diamond) and *H. pylori* positive (closed diamonds) from cohort 1. MtDNA relative values was measured as previously described according to nuclear DNA (nDNA) (see Material and methods).³⁵ Each symbol corresponds to a single patient. Bars and numbers indicate geometric means. Data are obtained from measures done in triplicate in three independent experiments. **P*<0.05 ; ***P*<0.001.

### Material and methods

### Study population.

Two cohorts of Mexican adult patients were studied. Cohort 1 included 48 healthy blood-donors as the reference group, 28 patients with non-atrophic gastritis (NAG), and 78 with gastric cancer (GC) for a total of 154 adults recruited during the period 2009-2011. The healthy individuals were blood donors recruited at the blood bank of the Instituto Mexicano del Seguro Social (IMSS), Medical Center SXXI. Cohort 2 included 48 patients with NAG, 34 patients with intestinal metaplasia (IM), and 49 with gastric cancer (GC) for a total of 131 patients recruited during the period 1999-2002. Patients from both cohorts were adults who were attended for gastroduodenal diseases at the Instituto Mexicano del Seguro Social (IMSS) Medical Center SXXI, in Mexico City. The inventors selected patients who were not under treatment for cancer and who had not been treated with antibiotics, bismuth compounds, proton pump inhibitors and non-steroidal anti-inflammatory drugs for at least two weeks prior to the study. Diagnosis was based on endoscopic examination and histopathology studies. ³⁰ All patients and controls were informed about the study and asked to sign a consent letter. The study was approved by the ethical committee from the National Council for Research on Health, IMSS.

### Collection of clinical samples, histological analysis and DNA extraction

For patients with gastritis or precancerous lesions, gastric biopsies collected from both the antrum and the corpus were taken. Biopsies were processed either for histology or immediately frozen at -70 °C. For histology, biopsies were immersed in formalin and processed for H&E staining for diagnosis of the lesions. Presence of non-atrophic gastritis, atrophic gastritis, intestinal metaplasia, or dysplasia was documented in each biopsy. The presence of *H. pylori* bacteria was confirmed by Giemsa staining. For patients with gastric cancer one fraction from the tumor lesion and one from the adjacent tissue were collected during surgery and divided for either histology or DNA extraction. For histology, tissues were immersed in formalin and processed for staining with H&E and analysed for *H. pylori* and for type of gastric tumor.

Circulating DNA was prepared from a sample of 10 ml of peripheral blood from each patient, and mononuclear cells were purified by centrifugation through a Ficoll-Hypaque density gradient. DNA was isolated from these cells using the salting-out microtechnique and frozen at -70°C until tested for mtDNA. The serum fraction was frozen at -20°C until tested for serology to *H. pylori* antigens.

### ELISA for IgG against H. pylori antigens.

IgG antibodies against *H. pylori* whole-cell antigens and against CagA *H*. *pylori* protein were tested in sera, using enzyme-linked immunoabsorbent assays which were previously validated by the inventors. ³¹

### Quantification of mitochondrial DNA by quantitative Polymerase Chain Reaction (q-PCR)

The quantification of mtDNA was performed as previously described ^{33, 35}, on total DNA isolated from circulating leucocytes using the StepOne^{™} Plus Real-Time PCR system and FastStart Universal SYBR Green Master (Applied Biosystems) following the manufacturer's instruction. MtDNA was quantified using a region in the *12SRNA* gene. The nuclear encoded *18SRNA* gene was used as an endogenous reference. Primers used to amplify the 12S and 18S gene sequences were: 12S (forward): 5'- GCT CGC CAG AAC ACT ACG AG; (reverse): 5'- CAG GGT TTG CTG AAG ATG GCG and 18S (forward): 5'- GAG AAA CGG CTA CCA CAT CC; (reverse): 5'- GCC TCG AAA GAG TCC TGT AT.³⁴ The qPCR was carried out in 20µl of total reaction volume containing 5µl of DNA (200 pg), 10µl of FastStart Universal SYBR Green Master, 0,2µL of primers (10µM) using an initial denature step at 95°C for 10 minutes and 40 cycles of 15 seconds at 95°C and 1 minute at 60°C. All samples were analyzed in triplicate. To determine the quantities of mtDNA and nDNA present in blood samples, the average threshold cycle number values (Ct) of the nDNA and mtDNA were obtained from each case. The level of mtDNA was calculated using the delta Ct (ΔCt) of average Ct of nDNA and mtDNA (ΔCt = Ct_{nDNA}-Ct_{mtDNA}) as 2^{ΔCt} as previously described. ³⁵

### Quantification of Interleukin 8 (IL-8) level in the serum

IL-8 levels were quantified in the serum of patients by enzyme-linked immunosorbent assay (ELISA) by using the ELISA kit BDOptEIA Set Human IL-8. Cat. No. 555244 commercialized by BD Biosciences according to the recommendations of the fabricant.

### Data analysis

The Mann-Whitney U test was used to compare mtDNA level in peripheral blood between healthy subjects and patients at various stages of the gastric pathologies, as well as to compare mtDNA level isolated from gastric biopsies. Differences were considered significant when P<0.05. For Odd Ratio (OR) determination the 95% confidence intervals were determined according to the Woolf's method (Woolf B, Ann Hum Genet, 1955, 19: 251-253).

### Results

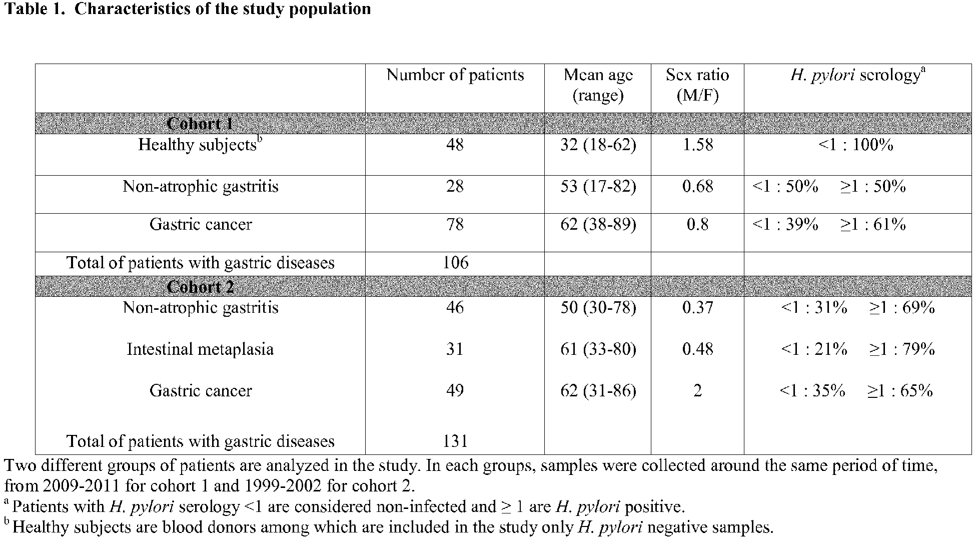

### Characteristics of the studied cohorts

Table 1 describes the general characteristics of the patients included in this study. In the cohort 1, half of the non-atrophic gastritis (NAG) patients were *H. pylori*-positive compared to 61% in the gastric cancer (GC) group. In the control group, samples are from blood donors, all *H. pylori* negative. Among GC patients, 28 were diagnosed as diffuse type with 9 cases inoperable and 6 with metastasis. Twenty GC were of intestinal type with 8 cases inoperable, 7 with hyperplasia and 7 with metastasis. In the cohort 2, 69% of the NAG patients are *H. pylori* positive compared to 75% and 65% in IM and GC groups. Overall, IM and GC patients were older with a mean age of 61 and 62 respectively, compared to 50 for NAG patients as also observed for cohort 1.

### Levels of MtDNA in peripheral leucocytes from patients with gastritis and gastric cancer

In cohort 1, mtDNA was quantified in DNA isolated from leucocytes of patients with non-atrophic gastritis (NAG) (n=28) and gastric cancer (n=78) and compared with healthy *H. pylori* negative subjects (n=48). The mean mtDNA value in the circulating blood white cells was 3-fold lower in patients with NAG *(p*=*0.02)* and slighlty higher 1.4-fold (p=0.0013) in GC patients compared to healthy subjects (Figure 1A). The comparison between NAG and GC patients showed a mean mtDNA level 4-fold higher in GC samples *(P*=*0.0009).* Among GC samples, 31 and 20 were diffuse and intestinal type, respectively. In both cases, mtDNA level was comprised between 1 and more than 30, compared to NAG samples found between 0 and less than 30 (Figure 1B). Also when comparing GC samples of all types, according to the presence of hyperplasia, metastasis or judged as inoperable by surgeons, no significant differences were observed on the variations of mtDNA level between the different GC groups. As reported in the table 1, the *H. pylori* serology was positive for half of the NAG and 61% of GC patients. No significant difference was observed comparing circulating blood white cells mtDNA level in *H. pylori*-positive and negative patients either both groups of patients (Figure 4). According to their mtDNA level, samples can be classified in 3 main groups corresponding to mtDNA<2 (Group I), 2<mtDNA<20 (Group II) and mtDNA>20 (Group III) (Figure 1C). The distribution of samples among these groups was not influenced by age or gender of the studied-population (data not shown), and showed important differences according to the gastric pathologies. In the Group I with mtDNA<2 no healthy subjects were detected compared to 46% NAG and 14% GC samples. Indeed, for all healthy subjects mtDNA levels were found in Group II comprised between a value of 2 to 20. Whereas for NAG and GC patients, the distribution of mtDNA level was broader with samples in the Groups I and II for NAG and I to III for GC. Furthermore, samples with mtDNA<1 corresponded mainly to NAG 39% (OR= 4.28 ; 95% CI=2.11-8.69) with only 2% GC (OR= 0.14; 95% CI=0.03-0.64) (Figure 1 D). MtDNA values lowest than 0.5 concerned only NAG samples (Figure 1 D). In addition, mtDNA levels >10 were more associated with NAG (OR=2.33 ; 95% CI=0.69-7.81) and GC (OR=5.13; 95% CI=1.95-13.48) than healthy subjects. Highest levels of mtDNA>30 were only observed in GC patients (Figure 1A).

### Comparison of serological levels of IL-8 in NAG and GC patients

Chronic inflammation is associated with atrophic gastritis and plays an important role at early steps during the promotion of carcinogenesis³⁶. In order to further improve the significance of the measure of circulating mtDNA regarding the inflammatory background of the patient, the level of the pro-inflammatory cytokine IL-8 in the serum was measured in 22 NAG and 77 GC patients from cohort 1. As reported in Figure 2A, the mean IL-8 value was 4-fold lower in NAG compared to GC patients. All NAG patients showed IL-8 levels lower than 50 pg/ml. A significant correlation was observed between progressive increase of IL-8 and peripheral leucocytes mtDNA levels for NAG patients (r=0.586 ; *P*=*0.0041*). However this group concerned only 22 samples (Figure 2B). In GC patients, two groups of samples according to IL-8 levels can be distinguished with IL-8<50 and IL-8>100 pg/ml corresponding to 79% and 21% of patients respectively. It is to be noticed that for 93% of GC patients, IL-8 higher than 100pg/ml is associated with mtDNA level of Group I or II. No significant correlation was observed between IL-8 and peripheral leucocytes mtDNA levels in GC patients. In addition, there were no significant differences in IL-8 according to the different groups defined for mtDNA level (data not shown).

### Levels of circulating mtDNA in white blood cells of patients with pre-neoplastic lesions (IM) or GC.

In order to analyse if the levels of mtDNA varied according to the suggested clinical history of GC, mtDNA was quantified in NAG (n=46), IM (n=31) and GC (n=49) patients in a second series of samples (Cohort 2) (Table 1). Despite the fact that overall the mean mtDNA levels measured for samples from cohort 2 were lower than for cohort 1 was more recent, no significant differences were observed when comparing circulating mtDNA level between patients with NAG and patients with IM (Figure 3A). As observed in the cohort 1, levels of mtDNA in GC patients were 3-fold higher that those observed in patients with NAG *(P*=*0.02)* and IM *(P*=*0.002)*. When distribution of mtDNA levels were analyzed according to the gastric pathology, the inventors found that values higher than 2 were observed in 35% of GC patients, compared to 4% in patients with NAG (OR=11.69; 95% CI=2.52-54.22) and 10% in patients with IM samples (OR=4.96; 95% CI=1.31-18.72). Thus, peripheral leucocytes mtDNA levels were higher in GC than IM patients. According to the data obtained from the two cohorts, inventors' results showed that peripheral leucocytes mtDNA level shifted to higher values between gastric pre-neoplastic (NAG and IM) and neoplastic stages (GC), establishing a signature assessing the evolution of the disease, that may be used when monitoring the health status of monitored patients or individuals.

### References

1. Parkin DM, Bray F, Ferlay J, Pisani P. Global cancer statistics, 2002. CA CAncer J. Clin 2005;55:74-108.
2. Kamangar F, Dawsey SM, Blaser MJ, Perez-Perez GI, Pietinen P, Newschaffer CJ, Abnet CC, Albanes D, Virtamo J, Taylor PR. Opposing risks of gastric cardia and noncardia gastric adenocarcinomas associated with Helicobacter pylori seropositivity. J Natl Cancer Inst 2006;98:1445-52.
3. Roukos DH. Early-stage gastric cancer: a highly treatable disease. Annals of Surgical Oncology 2003;11:127-9.
4. Resende C, Thiel A, Machado JC, Rlstmäki A. Gastric cancer: Basic aspects. Helicobacter 2011;16:38-44.
5. Bornschein J, Rokkas T, Selgrad M, Malfertheimer P. Gastric cancer: Clinical aspects, Epidemiology and Molecular Background. Helicobacter 2011 ;16:45-52.
6. Blaser MJ, Atherton JC. Helicobacter pylori persistence: biology and disease. J. Clin Invest 2004;113:321-33.
7. Atherton JC. The pathogenesis of Helicobacter pylori-induced gastro-duodenal diseases. Annual Rev. Pathol. Mech. Dis. 2006;1:63-96.
8. Correa P. Human gastric carcinogenesis : a multistep and multifactorial process. First American Cancer Society Award Lecture on Cancer Epidemiology and Prevention. Cancer Research 1992;52:6735-40.
9. Zhou L, Sung, J.J., Lin. S.. A five-year follow-up study on the pathological changes of gastric mucosa after H. pylori eradication. Chinese Med. J. 2003;116:11-4.
10. Ley C, Mohar, A., Guarner, J.. Helicobacter pylori eradication and gastric preneoplastic conditions: a randomized, double-blind, placebo-controlled trial. Cancer Epidemiol Biomarkers 2004;13:4-10.
11. Nozaki K, Shimizu N, Ikehara Y, Inoue M, Tsukamoto T, Inada K, Tanaka H, Kumagai T, Kaminishi M, Tatematsu M. Effect of early eradication on Helicobacter pylori-related gastric carcinogenesis in Mongolian gerbils. Cancer Sci 2003;94:235-9.
12. Lee CW, Rickman B, Rogers AB, Ge Z, Wang TC, Fox JG. Helicobacter pylori eradication prevents progression of gastric cancer in hypergastrinemic INS-GAS mice. Cancer Res 2008;68:3540-8.
13. Czarnecka. AM, Gammazza. AM, Di Felice. V, Zummo. G, Cappello.F. Cancer as a mitochondriopathy. J. Can Mol. 2007;3:71-9.
14. Clay Montier LL, Deng JJ, Bai Y. Number matters: control of mammalian mitochondrial DNA copy number J. Genet. Genomics 2009;36:125-31.
15. Lievre A., Chapusot C., Bouvier A, Zinzindohoue F, Piard Fea. J. Clin. Oncol 2005;23:3517-25.
16. Kamalidehghan B, Houshmand M, Shafa Shariat Panahi M, Rezza Abbaszadegan M, Ismail P, Bagher Shiroudi M. Tumoral cell mtDNA approximately 8.9kb deletion is more common than other deletions in gastric cancer. Arch. Med. Res. 2006;37:848-53.
17. Massimo V, et al. Microsatellite instability, mitochondrial DNA large deletions and mitochondrial DNA mutations in gastric carcinoma. Genes, Chromosomes and Cancer 2001;32:136-43.
18. Hiyama T, Tanaka S, Shima H, Kose K, Kitadai Y, Ito M, Sumii M, Yoshihara M, Shimamoto F, Haruma K, Chayama K. Somatic mutation of mitochondrial DNA in Helicobacter pylori-associated chronic gastritis in patients with and without gastric cancer. Int J Mol Med 2003;12:169-74.
19. Machado A, Figuereido C, Touati E, Maximo V, Sousa S, Michel V, Carneiro F, Nielsen FC, Seruca R, Rasmussen LJ. Helicobacter pylori infection downregulates nuclear and mitochondrial DNA repair in gastric cells. Clinical Cancer Res 2009;15:2995-3002.
20. Touati E. When bacteria become mutagenic and carcinogenic: lessons from H. pylori. Mutation Research/ Genetic Toxicology and Environmental Mutagenesis 2010;703:66-70.
21. Mambo. E, Chatterjee. A, Xing J, Tallini G, Haugen BR, Yeung S-C, Sukumar S, Sidransky D. Tumor-specific changes in mtDNA content in human cancer. Int J Cancer 2005;116:920-4.
22. Yu M. Generation, function and diagnostic value of mitochondrial DNA copy number alterations in human cancers. Life Sciences 2011;89:65-71.
23. Lee HC, Yin PH, Lin JC, Wu CC, Chen CY, Wu CW, al e. Mitochondrial genome instability and mtDNA depletion in human cancers. Ann N Y Acad Sci 2005;1042:109-22.
24. Ellinger J, Muller DC, Muller SC, Hauser S, Heukamp LC, von Ruecker A, Bastian PJ, Walgenbach-Brunagel G. Circulating mitochondrial DNA in serum: a universal diagnostic biomarker for patients with urological malignancies. Urol. Oncol. 2011.
25. Shen J, Platek M, Mahasneh A, Ambrosone CB, Zhao H. Mitochondrial copy number and risk of breast cancer: a pilot study. Mitochondrion 2010;10:62-8.
26. Qu F, Liu X, Zhou F, Yang H, Bao G, He X, Xing J. Association between mitochondrial DNA content in leukocytes and colorectal cancer risk: a case-control analysis. Cancer 2011;117:3148-55.
27. Bonner MR, Shen M, Liu CS, Divita M, He X, Lan Q. Mitochondrial DNA content and lung cancer risk in Xuan Wei, China. Lung Cancer 2009;63:331-4.
28. Xia P, An H, Dang C, Radpour R, Kohler C, Fokas E, Engenhart-Cabillic R, Holzgreve W, Zhong X. Decreased mitochondrial DNA content in blood samples of patients with stage I breast cancer. BMC Cancer 2009;21:454.
29. Liao LM, Baccarelli A, Shu XO, Gao YT, Ji BT, Yang GZ, Li HL, Hoxha M, Dioni L, Rothman N, Zheng W, Chow WH. Mitochondrial DNA copy number and risk of gastric cancer: a report from the Shangai women's health study. Cancer Epidemiol Biomarkers Prev 2011;20:1944-9.
30. Camorlinga-Ponce M, Flores-Luna L, Lazcano-Ponce E, Herrero R, Bernal-Sahagun F, Abdo-Francis JM, Aguirre-Garcia J, Munoz N, Torres J. Age and severity of mucosal lesions influence the performance of serologic markers in Helicobacter pylori-associated gastroduodenal pathologies. Cancer Epidemiol Biomarkers Prev 2008;17:2498-504.
31. Camorlinga-Ponce M, Torres J, Perez-Perez G, Leal-Herrera Y, Gonzalez-Ortiz B, Madrazo de la Garza A, Gomez A, Munoz O. Validation of a serologic test for the diagnosis of Helicobacter pylori infection and the immune response to urease and CagA in children. Am J Gastroenterol 1998;93:1264-70.
32. Zuchner S, Mersiyanova IV, Muglia M, Bissar-Tadmouri N, Rochelle J, Dadali E.L., Zappia M, Nelis E, Patitucci A., Senderek Jea. Mutations in the mitochondrial GTPase mitofusin2 cause Charcot-Marie-Tooth neuropathy type 2A. Nat Genet 2004;36:449-51.
33. Chatre L, Ricchetti M. Large heterogeneity of mitochondrial DNA transcription and initiation of replication exposed by single-cell imaging. J Cell Sci 2012.
34. Parone PA, Da Cruz. S, Tondera. D, Mattenberger. Y., James. D.I, Maechler P, Barja F, Martinou JC. Preventing mitchondrial fission impairs mitochondrial function and leads to loss of mitochondrial DNA. PLoS One 2008;3:s.
35. Fan AX, Radpour R, Haghighi MM, Kohler C, Xia P, Hahn S, Holzgreve W, Zhong XY. Mitochondrial DNA content in paired normal and cancerous breast tissue samples from patients with breast cancer. J Cancer Res Clin Oncol 2009;135:983-9.
36. Correa P. Human gastric carcinogenesis : a multistep and multifactorial process. First American Cancer Society Award Lecture on Cancer Epidemiology and Prevention. Cancer Res 1992;52:6735-40.

## Claims

1. An *in vitro* method for investigating the level of mtDNA in a biological sample removed from a patient susceptible of suffering from a gastric cancer condition or susceptible of suffering of condition(s) susceptible to evolve in a gastric cancer condition by pooling said biological sample into categories, said method comprising the steps of:
a. determining the level of mtDNA in said biological sample, and
b. comparing the level of mtDNA determined in step a) with a normal threshold value determined for healthy subject(s), and
c. from the comparison made in step b), assigning the tested biological sample to one of the following categories:
- Group I: if the level of mtDNA determined in step a) is decreased with respect to the normal threshold value introduced in step b) by less than 2 folds,
- Group II: if the level of mtDNA determined in step a) is increased from 2 to 20 folds with respect to the normal threshold value introduced in step b),
- Group III: if the level of mtDNA determined in step a) is increased with respect to the normal threshold value introduced in step b) by more than 20 folds.

2. The method of claim 1, for monitoring or diagnosing the health status of a patient susceptible of suffering from a gastric cancer condition or susceptible of suffering of condition(s) susceptible to evolve in a gastric cancer condition, said method further comprising the following step:
d. From the assignment to a Group I, II or III, made in step c), concluding about the health status of a patient from which the tested biological sample has been removed.

3. The method of any one of claim 1 or 2, for monitoring or detecting a risk of gastric carcinogenesis in a patient, wherein a risk of gastric carcinogenesis exists if the biological sample removed from said patient pertains to Group I or Group III.

4. The method of claims 2 or 3, wherein if the tested biological sample pertains to Group I determining whether the mtDNA level is less than 0.5 fold with respect to normal threshold value, and, if so, concluding about the existence of a risk of gastric carcinogenesis at an early stage, in particular a pre-neoplasic stage, for example at a gastritis stage.

5. The method of claims 2 or 3, wherein if the tested biological sample pertains to Group III determining whether and the mtDNA level is more than 30 folds and, if so, concluding about the existence of a gastric cancer.

6. The method of any one of claims 2 to 5, which comprises a previous, simultaneous or parallel step a detection of an *Helicobacter pylori* infection, in particular through detection of antigen(s) specific for *H*. *pylori* infection, or through an assay involving DNA amplification and subsequent detection of said DNA, or detection of the presence of specific *H. pylori* IgA and IgG antibodies in a biological sample removed from the tested patient, or through an ¹³C urea breath test performed on the tested patient

7. The method of any one of claims 1 to 6, wherein the biological sample is from a patient diagnosed with gastric carcinogenesis and under treatment for this condition or not, and/or a patient having an ongoing, treated or not, *Helicobacter pylori* infection, and/or a patient having antecedents of *Helicobacter pylori* infection(s), eradicated or not, and/or an individual having gastric pain and/or a family history of gastric cancer.

8. The method of any one of claims 1 to 7, wherein the level of mtDNA is determined by quantitative polymerase chain reaction (q-PCR) following the steps of:
- preparing the biological sample to provide access to the nucleic acid, especially mitochondrial nucleic acid of cells;
- contacting the prepared sample with oligonucleotide primers targeting the mtDNA;
- performing amplification cycles,
- simultaneously running amplification of a normalizer nDNA,
- quantitatively detecting the mtDNA and the normalizer nDNA.
- determining the level of mtDNA through reference to a selected normalizer nDNA sequence, the level of mtDNA being calculated according to the formula 2^{ΔCt}, wherein ΔCt = Ct_{nDNA} - Ct_{mtDNA}.

9. The method of any one of claims 1 to 8, wherein the biological sample removed from the patient is a blood sample or a biopsy sample, in particular a biopsy sample of gastric mucosa.

10. The method of any one of claims 1 to 9, wherein the level of mtDNA is determined by testing circulating blood mtDNA, in particular is determined by testing the mtDNA of leukocyte(s).

11. A method for *in vitro* monitoring or diagnosing the health status of a patient susceptible of suffering from a gastric cancer condition or susceptible of suffering of condition(s) susceptible to evolve in a gastric cancer condition, said method comprising the following steps on a biological sample removed from said patient:
a. Performing a method according to any one of claims 1 to 10 for investigating the level of mtDNA in the tested patient, and
b. Measuring, in parallel to the investigation of the level of mtDNA of step a., the level of cytokine IL-8 in a biological sample removed from the patient tested in step a, and
c. Concluding about the health status of a patient on the basis of the results obtained from step a. and b.

12. The method of claim 11, for detecting a risk of presence of a gastric cancer in a tested patient comprising determining whether the level of mtDNA pertains to Group I or Group II and determining whether the level of cytokine IL-8 is above 100 pg/mL.

13. A method for *in vitro* monitoring or diagnosing the health status of a patient susceptible of suffering from a gastric cancer condition or suffering from a gastric cancer condition or susceptible of suffering of condition(s) susceptible to evolve in a gastric cancer condition, said method comprising the following steps:
a. Performing a method according to any one of claims 1 to 10 for investigating the level of mtDNA in the tested patient, and
b. Measuring, in parallel to the investigation of the level of mtDNA of step a., the level of any one of the following parameters: cytokine IL-8, platelet count (PLT) and/or mean platelet volume (MPV) and/or percentage of large platelets (LPLT), inflammatory markers such as IL-6 and/or IL-23, or any combination of these parameters in a biological sample removed from the patient tested in step a., and
c. Concluding about the health status of a patient on the basis of the results obtained from step a. and b.

14. Kit suitable for carrying out a method as defined in any one of claims 1 to 13, comprising:
- At least one pair of specific oligonucleotide primers specific for hybridization with mtDNA and, optionally, at least one pair of specific oligonucleotide primers specific for hybridization with *H. pylori* nucleic acid(s) sequence(s), and, optionally, one or several of the following reagents,
- nucleotides (e.g. dATP, dCTP, dGTP, dUTP),
- a DNA polymerase, in particular a thermostable DNA polymerase, such as a Taq DNA Polymerase,
- at least one dye for staining nucleic acids, in particular a dye detectable in a real-time PCT equipment,
- optionally, a buffer solution,
- optionally, reagents necessary for the hybridation of the primers to their targets,
- optionally, a reference dye and,
- a notice providing instructions for use and expected values for interpretation of results.

15. Kit suitable for carrying out a method as defined in any one of claims 1 to 13, comprising:
- at least one antibody specific for a protein selected amongst: IL-8, IL-6, IL-23 or a combination of several antibodies specific for IL-8, IL-6, IL-23, and, optionally, at least one antibody specific for *H. pylori* antigen(s), such as CagA antigens, and, optionally one or several of the following reagents,
- a secondary antibody or reagent to reveal a complex between specific antibody(ies) recited above and its(their) target,
- optionally, a buffer solution,
- optionally, an assay plate, and
- optionally a notice providing instructions for use and expected values for interpretation of results.

16. Use of a kit according to any one of claims 14 or 15 for investigating the level of mtDNA in a biological sample and/or of the level(s) of any one of the following parameters: IL-8, IL-6, IL-23 or combination(s) thereof, and/or monitoring or diagnosing the health status of a patient susceptible of suffering from a gastric cancer condition or susceptible of suffering of condition(s) susceptible to evolve in a gastric cancer condition and/or monitoring or detecting a risk of gastric carcinogenesis, or suffering from a gastric cancer condition, including the health status of said patient with respect to an *H. pylori* infection.
